# EUROPEAN PATENT SPECIFICATION

(11) **EP 2 538 845 B1**
(45) Date of publication and mention of the grant of the patent: **31.08.2016**
(21) Application number: 11714707.4
(22) Date of filing: 18.02.2011
(51) Int. Cl.: A61B 6/14, A61C 19/04, A61C 1/08, A61C 5/02, A61C 17/02

(54) **3D DIGITAL ENDODONTICS**
DIGITALE 3D-ZAHNWURZELABBILDUNGEN
ENDODONTIE NUMÉRIQUE 3D

(30) Priority: 18.02.2010 GB 201002778
(43) Date of publication of application: 02.01.2013
(73) Proprietor: Dentsply Implants NV, 3500 Hasselt (BE); Maillefer Instruments Holding S.À.R.L., 1338 Ballaigues (CH)
(72) Inventor: VAN LIERDE, Carl, B-9420 Meerbeke (BE); VALLOTTON, Paul-Henri, CH-1142 Pampigny (CH)
(74) Representative: Mayjonade, Bernard
(86) International application number: PCT/EP2011/052457
(87) International publication number: WO 2011/101447

(56) References cited:
- EP-A1- 1 547 544
- EP-A1- 1 563 803
- WO-A1-2009/116650
- CA-A1- 2 484 475
- DE-A1- 4 404 983
- DE-C- 399 564
- FR-A- 1 602 331
- JP-A- 2011 030 637
- US-A- 4 708 651
- US-A1- 2006 110 710
- US-A1- 2009 111 068
- US-A1- 2010 304 326
- US-B1- 6 390 814
- BERGMANS ET AL: "A methodology for quantitative evaluation of root canal instrumentation using microcomputed tomography.", INTERNATIONAL ENDODONTIC JOURNAL, vol. 34, no. 5, 1 July 2001 (2001-07-01), pages 390-8, XP55001535, ISSN: 0143-2885

## Description

### Field of the invention

The invention relates to a 3D digital endodontics system and method, characterized in that 3D imaging equipment are used to digitize the infected tooth or teeth and wherein a 3D representation of the root canal system is extracted from the image data and visualized on a computer screen. The invention further relates to a surgical template designed to localize the root canal intra-operatively.

### Background

Endodontics is a specialist field of dentistry that deals with surgical and therapeutic procedures for either protecting the tooth pulp or removing it from the root canals when it has been injured or is diseased. The pulp contains nerves, arterioles and venules as well as lymphatic tissue and fibrous tissue, and when it dies or becomes necrotic, endodontic treatment is required.

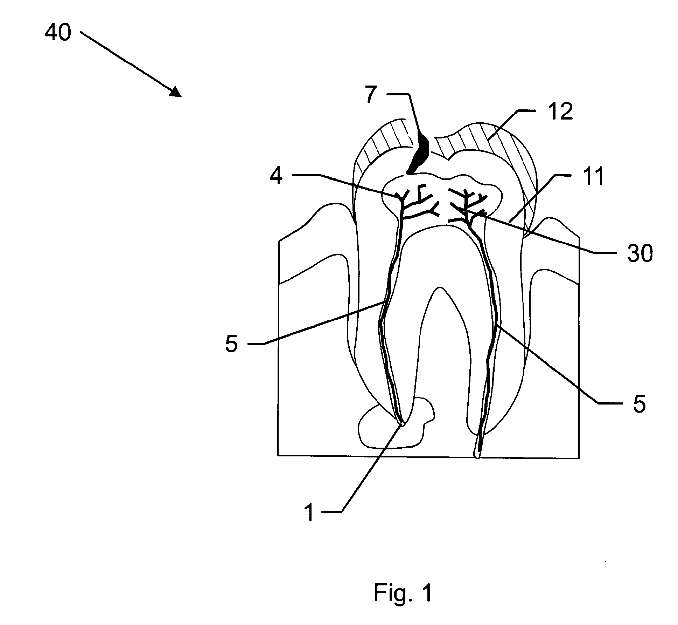

Fig. 1 shows a tooth 40 with enamel 12 one or more canals 5 with a root end opening 1 within the dentin 11 of the tooth connect up into the dental pulp chamber 4. Together they form a natural cavity that is filled with highly vascularized, loose connective tissue (i.e. the dental pulp tissue 30 comprising nerves and blood vessels). This tissue may become infected due to tooth decay (caries 7) or tooth fracture, allowing bacteria to access the pulp chamber 4 and/or the root canals 5.

Generally, the endodontic treatment comprises the following steps:
- Preparation of an access cavity by removing a substantial part of the occlusal surface of the tooth, removing the coronal pulp and enlarging the pulp chamber and root canal orifice(s),
- Exploration of the root canal, in particular to assess the canal length and to extract the radicular pulp,
- Mechanical shaping of the root canal with a sequence of instruments,
- Cleaning and disinfection of the root canal by means of irrigation,
- Obturation by filling the root canal with a sealing material (usually gutta-percha).

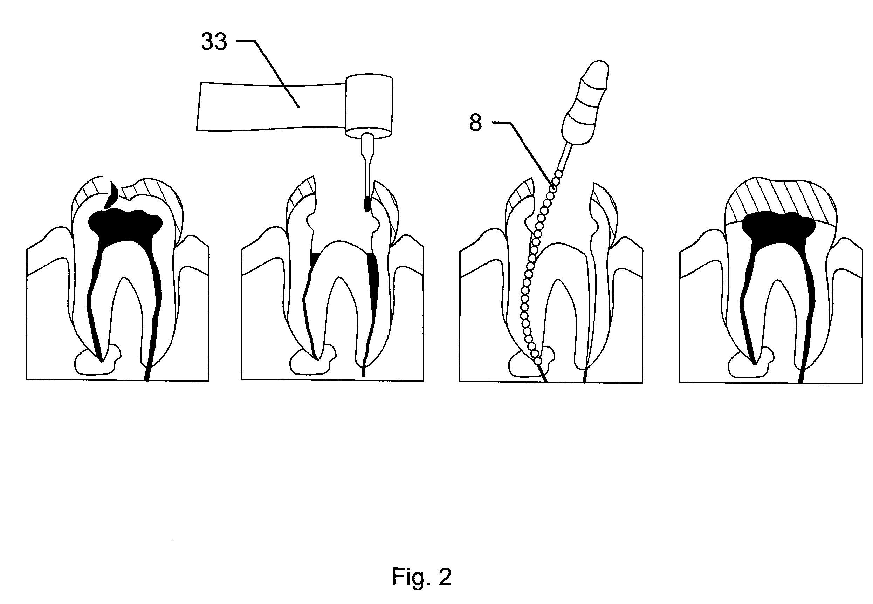

Specialized instruments used for endodontic treatment are shown in Fig. 2, such as endodontic files 8, micro-openers 33, etc.

### Treatment limitations

Efficacy of the endodontic treatment is highly dependent on the extent to which the diseased/infected tissue can be removed from the root canal system. This in turns depends on:

### 1. Relevance of information on the specific anatomy of a canal system

Today, X-ray radiographs are used by most of the practitioners to assess the extent of the disease as well as the tooth anatomy. However the inherent two-dimensional nature of radiographs (which are planar projections of 3D objects) can lead to surgical mistakes due to incorrect image interpretation and/or insufficient anatomical information. Typical root canal treatment mistakes due to deficient anatomical information include:
- missing the treatment of a main root canal (for instance the 4^{th} "mesio-buccal 2" canal in a top molar_tooth),
- missing the treatment of accessory lateral canals,
- missing the detection of root fractures and cracks,
- damaging the canal(s) by creating apical zips, ledges, or perforations due to unforeseen sharp canal curvatures, furcations, and other misleading anatomical features.

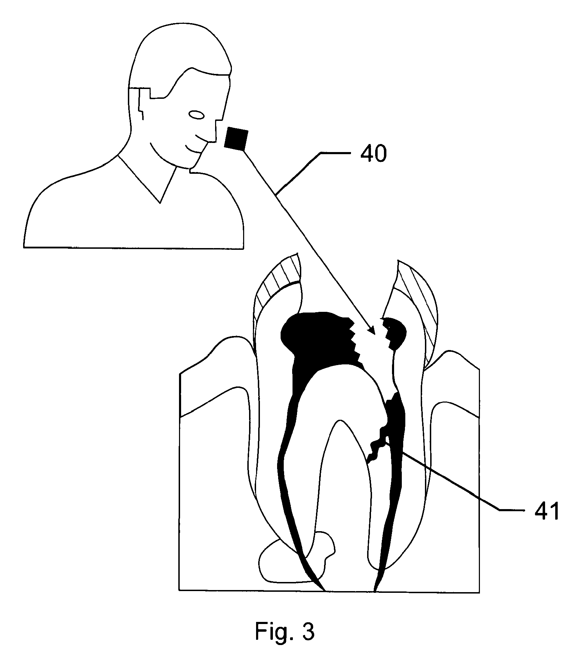

The use of special equipment such as microscopes does not solve the problem of insufficient anatomical information. For instance, as shown in Fig. 3, accessory lateral root canals 41 cannot be seen due to a missing line-of-sight 40. If an infected canal is missed, it may cause continued infection or "flare up" of the tooth even after treatment. Similar problems can occur with oddly shaped root canals.

### 2. Adequacy of the instrument set (shape, size, length, sequence) selected to perform the root canal shaping

Another difficulty during endodontic treatment is the accurate determination of the length of the root canal. If the dentist fails to reach the apex of the root canal, decayed pulp may remain present and jeopardise the success of the treatment. Conversely, if the root canal shaping is performed beyond the apex, healthy bone tissue may be destroyed and problems may occur when trying to seal the root canal. Electronic equipment based on impedance measurements exists to assess the depth of penetration of the endodontic instruments intra-operatively ("apex locators"). However this technique lacks precision and does not mechanically limit the penetration. Another technique exists in which the endodontic instruments are equipped with physical stops (usually plastic rings). This technique is however only partly reliable because it depends on the way the stops abut against the irregular occlusal surface of the tooth. In all cases, information on the length of the root canal measured in 3D remains unavailable.

Also missing is information on the dimensions and shape of the root canal cross section at various levels between the orifice and the apex. This prevents a specific selection of the instruments based on the adequacy of their shape and dimensions for optimized canal shaping and removal of diseased tissue.

### 3. Ability to perform exhaustive root canal irrigation and disinfection

Another problem associated with current practices in endodontics is related to the irrigation of the root canal. The irrigation plays a key role in disinfecting and debriding the canal system subsequent to the mechanical cleaning. It serves as a chemical preparation of the root canal for the purposes of lubricating the canal, dissolving pulp remnants, washing out debris, killing bacteria/micro-organisms and cleaning the smear layer attached to the inside of the canals. A typical irrigation fluid used is a solution of sodium hypochlorite in water.

Two factors influence the effectiveness of the irrigation:
- contact of the irrigation fluid with the canal walls,
- stirring of the solution during irrigation.

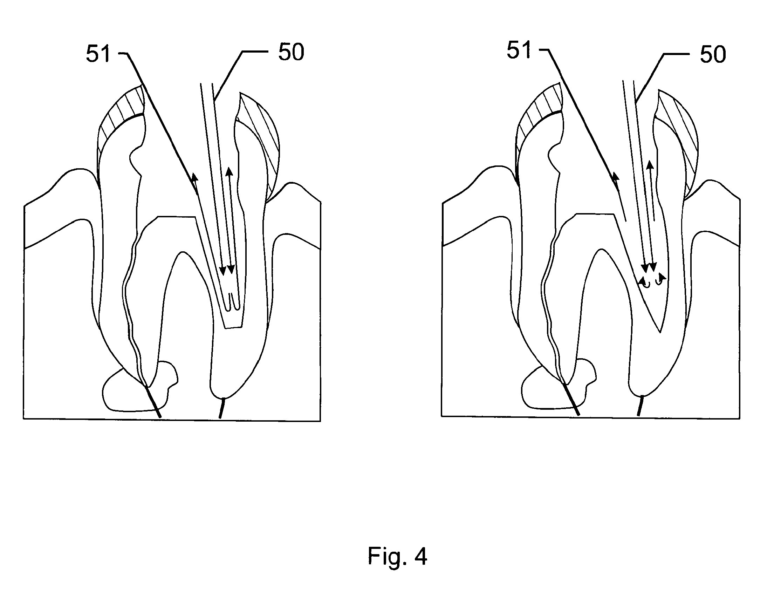

The first factor depends on the adequacy between instrument and canal shape (see above). The second factor however is determined by the geometrical confinements of the canals themselves. Fig. 4 shows that as long as the width of the canals is sufficient to allow for in 50- and outflow 51 of the irrigation solution, an adequate stirring is to be expected. However once the canals become too narrow, the irrigation fluid merely tops off the canal and is no longer refreshed over the entire length of the canal. Even sonic/ultrasonic methods fail to solve this problem since they merely agitate the solution locally in the canals.

The publication "A methodology for quantitative evaluation of root canal instrumentation using microcomputed tomography" by L Bergmans et al., International Endodontic Journal, volume 34, 390-398, 2001, discloses a method and system according to the respective preambles of claims 1 and 8.

### Summary of the disclosure

The present invention has as an object to avoid at least one of the following problems: avoiding mistakes by providing methods and systems with more precise, 3D information on the anatomy prior to the treatment, avoiding missing information on the dimensions and shape of the root canal cross section at various levels between the orifice and the apex, improving irrigation of the root canal.

According to the current disclosure a method and system are described for 3D digital endodontics characterized in that 3D imaging equipment such as a CT or MRI scanner, ultrasound or the like are used to digitize the infected tooth or teeth, a 3D representation of the root canal system is extracted from the image data and visualized on a computer screen. In a preferred embodiment a surgical template is designed to guide the endodontic instruments to localize the root canal(s) intra-operatively. In accordance with a further embodiment said template guide is manufactured by means of a computer driven system (e.g. milling, rapid prototyping, etc.).

In some embodiments a computer based method for 3D digital endodontics is described, 3D imaging equipment being used to digitize an image of an infected tooth or teeth to thereby form image data, the method comprising:
providing a user interface to extract a 3D representation of a root canal system from the image data and to visualize the root canal system on a visual display unit.

The method may also comprise manufacturing said template by means of a computer driven system, wherein the computer driven system is a milling, a rapid prototyping, or layered manufacture system.

Preferably the 3D imaging equipment is for generation of volumetric data such as a CT scanner, an MRI scanner, or an ultrasound scanner.

Capture images may be analysed using a segmentation technique for obtaining a 3D description of the root canal.

Preferably the user interface is adapted to allow 3D and cross-sectional views of the root canal on coronal, sagittal and/or transverse plans.

Digital templates of a plurality of root canal instruments are provided, which has the advantage of allowing 2D superimposed views of instruments and canal shapes or canal cross-sections for treatment planning and instrument selection.

In accordance with the method location of root canal orifices is determined by extracting the root canal system from the image data. The step of extracting can be carried out by indicating points along the axis of the root canal in one or multiple slices of the image.

The points defining the root canal can be connected and make up a 3D line graph representative of the root canal system of the tooth which allows for easy graphical presentation.

Advantageously, the orifice of each root canal is visualized in 3D as a point with a distinct indicator such as a colour.

The root canal orifice can be expressed as coordinates in a tooth specific coordinate system.

Preferably the user interface is adapted to allow design of a surgical template to guide endodontic instruments to localize the root canal intra-operatively. Such a template can be adapted to extend out onto gums and soft tissue or wherein the template is adapted to be supported on an occlusal surface of at least one of the infected teeth or adapted to be supported on an occlusal surface of one or more neighbouring teeth. For example, in the template one or more features to guide endodontic instruments used during a surgical intervention are introduced. The one or more features can be guidance tubes that help direct the endodontic tools to the orifices of the root canals, thereby allowing for a less invasive intervention. Alternatively the one or more features can provide physical depth control to the endodontic instruments.

The template preferably has a stop such as a flat or other surface on an non-supported side which acts as a physical stop for an endodontic instrument. The location of the physical stop canbe determined by means of a calculation comparing the length of the root canal as determined from the acquired 3D representation of the root canal and the length of the foreseen endodontic instrument, such that when inserted, the endodontic instrument penetrates the root canal at a desired depth. As an example, the endodontic instrument can be fitted with a flange to abut against said flat surface on the template. Such a flange can be hinged around a point on its longitudinal axis to assure a "face-to-face" connection at the interface with the template.

The one or more of said features can be located at the level of the gums, oblique or perpendicular to the axis of the infected tooth. The one or more features can be for guiding a dental bur or drill for making a lateral access hole through the bone surrounding the root of the infected tooth at the location of a lateral root canal that could not be cleaned via a typical occlusal access route through the pulp chamber. The one or more features can allow for irrigation fluid to be injected into the root canal via an apical root end opening.

Preferably, irrigation fluid is designed to flow from the apical end of the root canal up to the pulp chamber and further out of the tooth via an occlusal access opening.

The present disclosure also provides a surgical template having at least one guide for an endodontic instrument for localizing a root canal intra-operatively. The template can be adapted to be supported on an occlusal surface of at least one of the infected teeth or adapted to be supported on an occlusal surface of one or more neighbouring teeth. The template can be adapted to extend out onto gums and soft tissue.

Optionally, the template can have one or more features to guide endodontic instruments used during a surgical intervention. The one or more features can be guidance tubes that help direct the endodontic tools to the orifices of the root canals, thereby allowing for a less invasive intervention. The one or more features can provide physical depth control to the endodontic instruments. Preferably, the template can have a stop such as a flat or other surface on an non-supported side which acts as a physical stop for an endodontic instrument. The location of the physical stop can be determined by means of a calculation comparing the length of the root canal as determined from the acquired 3D representation of the root canal and the length of the foreseen endodontic instrument, such that when inserted, the endodontic instrument penetrates the root canal at a desired depth. The one or more of said features can be located at the level of the gums, oblique or perpendicular to the axis of the infected tooth. The one or more features can be for guiding a dental bur or drill for making a lateral access hole through the bone surrounding the root of the infected tooth at the location of a lateral root canal that could not be cleaned via a typical occlusal access route through the pulp chamber. The one or more features can allow for irrigation fluid to be injected into the root canal via an apical root end opening.

The present disclosure also provides a computer program product for execution on a computing system having a processor and memory, the computer program implementing any of the methods of the present disclosure as described above and the text below. The present disclosure also includes a computer readable signal storing medium, for non-transitorily storing the computer program.

The present disclosure also provides a computer based system for 3D digital endodontics, 3D imaging equipment being used to digitize an image of an infected tooth or teeth to thereby form image data, the system comprising:
a user interface to extract a 3D representation of a root canal system from the image data and visualized on a visual display unit.

The system can include means for gradient - based extraction of the root canal system out of the image data using the grey values, and/or means the root canal, optionally including lateral side channels, to be highlighted in the image data following use of a radiopaque dye which diffuses through the tissue in the root canal and subsequent CBCT imaging, and/or means for automatic identification and tracing of the volume of the infected / inflamed tissue based on the grey values.

The user interface can be adapted to allow design of a surgical template to guide endodontic instruments to localize the root canal intra-operatively.

The system may further comprise means for manufacturing said template by means of a computer driven system. The computer driven system is preferably a milling, a rapid prototyping, or layered manufacture system.

The 3D imaging equipment for use with the system and which used for generation of volumetric data is preferably a CT scanner, an MRI scanner, or an ultrasound scanner.

Means of segmentation of images is provided for obtaining a 3D description of the root canal system.

The user interface can be adapted to allow 3D and cross-sectional views of the root canal on coronal, sagittal and/or transverse plans. To assist, digital templates can be stored in a memory of a plurality of root canal instruments, further comprising means for allowing 2D superimposed views of instruments and canal shapes or canal cross-sections for treatment planning and instrument selection.

Means can be provided for location of root canal orifices by extracting the root canal system from the image data. The means for location can be adapted to indicate points along the axis of the root canal in one or multiple slices of the image. The points can be connected and make up a 3D line graph representative of the root canal system of the tooth. Preferably means for visualising the orifice of each root canal in 3D as a point with a distinct indicator such as a colour is provided. There can also be means for expressing the root canal orifice as co-ordinates in a tooth specific co-ordinate system.

### Brief description of the drawings

Further features of the present disclosure will become apparent from the drawings, wherein:
FIG. 1 is a schematic representation of a tooth and its anatomy.
FIG. 2 is a picture of specialized instruments found in the prior art used for endodontic treatment.
FIG. 3 is a representation of line-of-sight of a microscope used for obtaining anatomical information of a tooth.
FIG. 4 is a schematic representation of the in- and outflow of an irrigation solution used for root canal treatment.
FIG. 5a is a gradient-based extraction out of the images using the grey values according to an embodiment of the invention.
FIG. 5b is a CBCT imaging of the root canal system, according to an embodiment of the invention, using a radiopaque dye.
FIG. 5c is picture of the automatic identification and tracing of the volume of the infected or inflamed tissue based on the grey values.

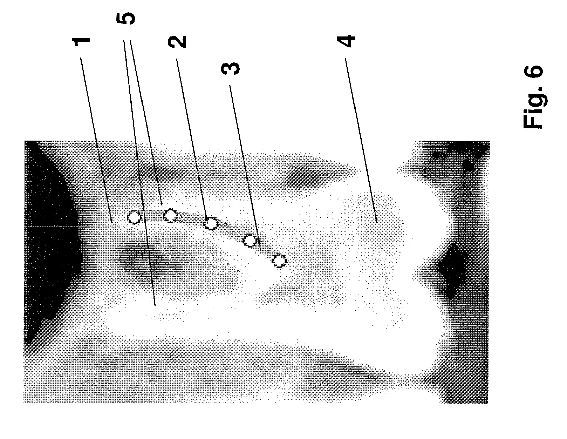

FIG. 6 is a picture illustrating the extracting the root canal system from an image data along the axis of the root canal in one or multiple slices of the image set.
FIG. 7 is a schematic representation of the surgical template according to a preferred embodiment of the disclosure.
FIG. 8a is a schematic representation of the surgical template according to an embodiment of the invention.
FIG. 8b is a schematic representation of the surgical template and irrigation method according to an embodiment of the disclosure
FIG. 9 is a schematic representation of a computing system which can be utilized with the methods described and in a system according to the present invention.

### Description of preferred embodiments

The present disclosure will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description it does not exclude other elements or steps. Where an indefinite or definite article is used when referring to a singular noun e.g. "a" or "an", "the", this includes a plural of that noun unless something else is specifically stated.The term "comprising", should not be interpreted as being restricted to the means listed thereafter; it does not exclude other elements or steps. Thus, the scope of the expression "a device comprising means A and B" should not be limited to devices consisting only of components A and B. It means that with respect to the present disclosure, the only relevant components of the device are A and B.Furthermore, the terms first, second, third and the like in the description are used for distinguishing between similar elements and not necessarily for describing a sequential or chronological order. It is to be understood that the terms so used are interchangeable under appropriate circumstances and that the embodiments of the disclosure described herein are capable of operation in other sequences than described or illustrated herein. Moreover, the terms top, bottom, over, under and the like in the description are used for descriptive purposes and not necessarily for describing relative positions. It is to be understood that the terms so used are interchangeable under appropriate circumstances and that the embodiments of the disclosure described herein are capable of operation in other orientations than described or illustrated herein.

In the drawings, like reference numerals indicate like features; and, a reference numeral appearing in more than one figure refers to the same element. The drawings and the following detailed descriptions show specific embodiments of a 3D digital endodontics system and method.

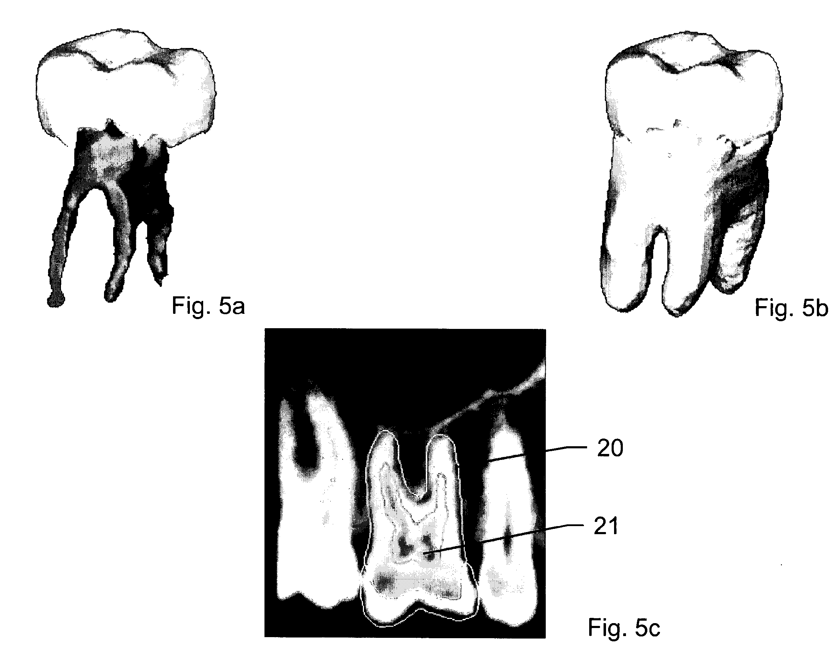

According to one aspect of the disclosure, segmentation techniques can be used to obtain a 3D description of the root canal system. A dedicated software program allows 3D and cross-sectional views of the canal on coronal, sagittal and transverse plans. Another feature of the disclosure is the integration of digital templates of various root canal instruments that will be integrated to said software, allowing the dentist to obtain 2D superimposed views of instruments and canal shapes (including cross-sections) for treatment planning and instrument selection purposes. The present invention also allows automatic detection and display of the root canal system as illustrated in Figures 5a-5c. Automatic detection and display of the root canal system can be done by a gradient-based extraction method as illustrated in Fig. 5a. The latter is based on the gradient-based extraction out of the images using the grey values. Another detection and display of the root canal system is illustrated in Fig. 5b where radiopaque dye is used which diffuses through the tissue in the root canal and subsequent CBCT imaging so the root canal, including e.g. lateral side channels are highlighted in the images. Automatic identification and tracing 20 of the volume of the infected/inflamed tissue 21 based on the grey values can also be performed as illustrated in Fig. 5c.

The present disclosure also provides a user interface which can provide, for example, suggestions (by the system) of the appropriate tools to use by virtue of the geometric characterization (e.g. 3D length and width) of the infected canal.

Hence, according to an aspect of the disclosure, the location of the root canal orifices 1 is determined by extracting the root canal 5 system from the image data is e.g. by indicating points 2 along the axis of the root canal in one or multiple slices of the image set which is shown on Fig. 2. The points are connected and make up a 3D line graph representative 3 of the root canal system of the tooth. The orifice 1 of each canal may be visualized in 3D as a point with a distinct marker such as a distinct color e.g. to distinguish it among the other points representative of the pulp chamber 4. The root canal orifice 1 can be expressed as coordinates in a user specified e.g. tooth specific coordinate system.

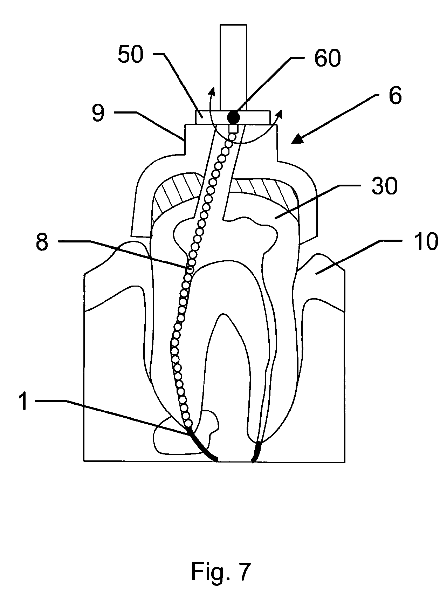

According to another aspect of the disclosure, a surgical template 6 as shown in Fig. 7 can be supported on the occlusal surface 30 of at least the one tooth being treated. Alternatively it may be supported on the occlusal surfaces of one or more neighboring teeth as well. The surgical template 6 may or may not extend out onto the gums and soft tissue 10. Incorporated in the template are one or more features to guide endodontic instruments used during the surgical intervention.

According to one embodiment, such features may be guidance tubes that help direct the endodontic tools (such as endodontic files 8) to the orifices 1 of the root canals, thereby allowing for a less invasive intervention.

According to a another embodiment, such features incorporated in the template may provide physical depth control to the endodontic instruments. The template has a physical stop for the endodontic instrument, e.g. a flat 9 or other surface on the non-supported side (typically the occlusal side) which acts as the physical stop for the endodontic instrument. The location of the stop surface is determined by means of a calculation comparing the length of the root canal (as determined from the acquired 3D representation of the root canal) and the length of the foreseen endodontic instrument, such that when inserted, the endodontic instrument 8 penetrates the root canal at the exact desired depth, typically until the apical end of the root canal. The endodontic instrument may be fitted with a flange 50 to abut against said flat surface on the template. The flange may hinge (e.g. like a ball joint) around a point 60 on its longitudinal axis to assure a "face-to-face" connection at the interface with the template 6.

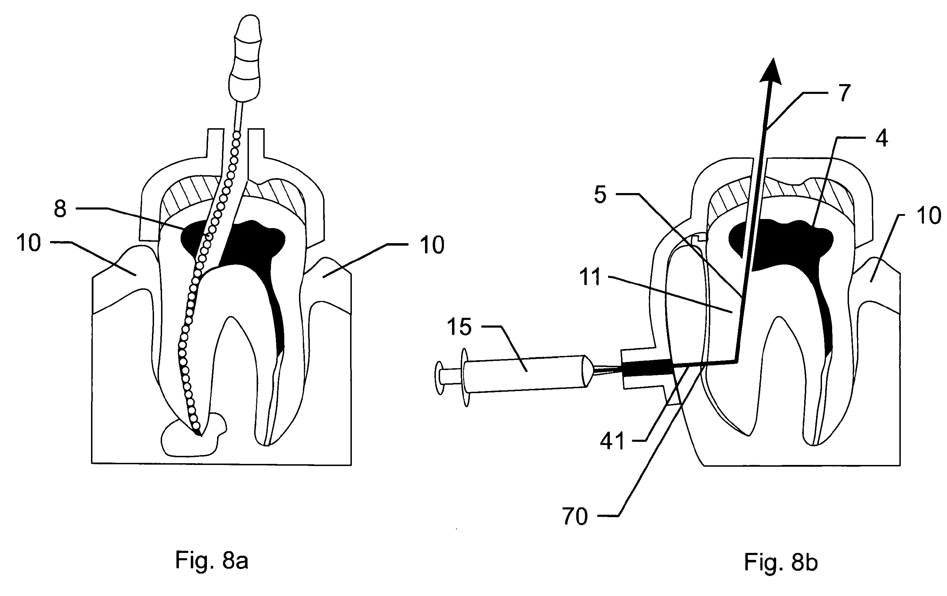

According to yet another embodiment, which is illustrated in Figs. 8a and 8b, one or more of said features may be located at the level of the gums 10, oblique or perpendicular to the axis of the tooth. The features could for instance guide a dental bur/drill for making a lateral access hole 70 through the bone 11 surrounding the root of the tooth 5 at the location of a lateral root canal 41 that could not be cleaned via a typical occlusal access 7 route through the pulp chamber 4. Similarly the feature could allow for irrigation fluid 15 to be injected into the root canal via the apical root end opening 11. Irrigation fluid 15 would then flow from the apical end of the root canal up to the pulp chamber 4 and further out of the tooth via an occlusal access opening 7. This assures sufficient volume exchange to thoroughly rinse, disinfect and clean the entire root canal system.

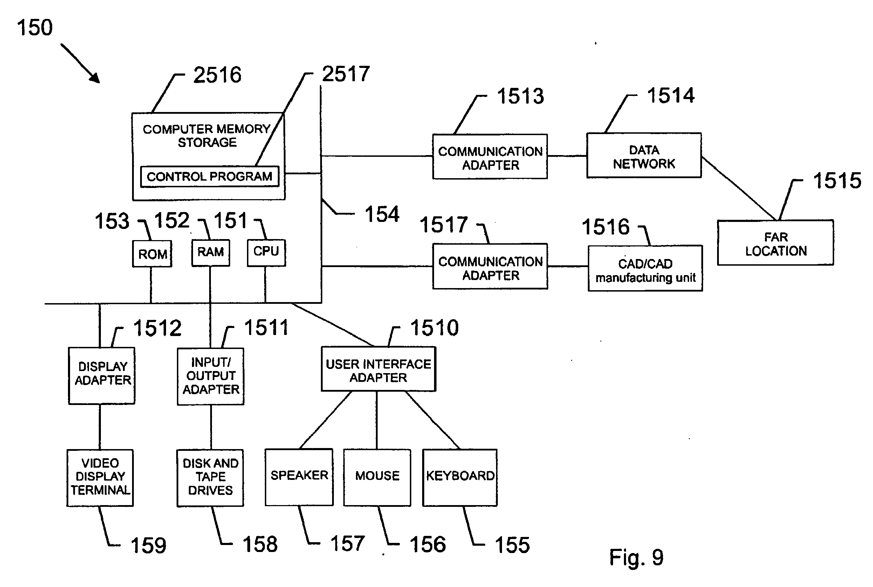

Fig. 9 is a schematic representation of a computing system which can be utilized with the methods and in a system according to the present invention including computer programs such as 3-matic™ as supplied by Materialise N.V., Leuven, Belgium. A computer 150 is depicted which may include a video display terminal 159, a data input means such as a keyboard 155, and a graphic user interface indicating means such as a mouse 156. Computer 150 may be implemented as a general purpose computer, e.g. a UNIX workstation or a personal computer.

Computer 150 includes a Central Processing Unit ("CPU") 151, such as a conventional microprocessor of which a Pentium processor supplied by Intel Corp. USA is only an example, and a number of other units interconnected via bus system 154. The bus system 154 may be any suitable bus system the above figure is only schematic. The computer 150 includes at least one memory. Memory may include any of a variety of data storage devices known to the skilled person such as random-access memory ("RAM"), read-only memory ("ROM"), and non-volatile read/write memory such as a hard disc as known to the skilled person. For example, computer 150 may further include random-access memory ("RAM") 152, read-only memory ("ROM") 153, as well as a display adapter 1512 for connecting system bus 154 to a video display terminal 159, and an optional input/output (I/O) adapter 1511 for connecting peripheral devices (e.g., disk and tape drives 158) to system bus 154. Video display terminal 159 can be the visual output of computer 150, which can be any suitable display device such as a CRT-based video display well-known in the art of computer hardware. However, with a desk-top computer, a portable or a notebook-based computer, video display terminal 159 can be replaced with a LCD-based or a gas plasma-based flatpanel display. Computer 150 further includes user interface adapter 1510 for connecting a keyboard 155, mouse 156, and optional speaker 157. The relevant data describing the 3-D object to be formed may be input directly into the computer using the keyboard 155 or from storage devices such as 158, after which a processor carries out a method in accordance with the present disclosure. The results of the method may be transmitted to a further near or remote location, e.g. a CAD/CAM processing facility to manufacture an endodontic template in accordance with the details provided by computer 150.

A CAD/CAM manufacturing unit 1516 may also be connected via a communications adapter 1517 to bus 154 connecting computer 150 to a data network such as the Internet, an Intranet a Local or Wide Area network (LAN or WAN) or a CAN. The manufacturing unit 1516 may receive a template descriptor file directly from computer 150 running a computer program for design of endodontic templates in accordance with the present disclosure or a value or descriptor file derived from such an output of computer 150. Alternatively, the unit 1516 may receive the relevant design data indirectly on a suitable signal storage medium such as a diskette, a replaceable hard disc, an optical storage device such as a CD-ROM or DVDROM, a magnetic tape or similar.

Computer 150 also includes a graphical user interface that resides within machine-readable media to direct the operation of computer 150. Any suitable machine-readable media may retain the graphical user interface, such as a random access memory (RAM) 152, a read-only memory (ROM) 153, a magnetic diskette, magnetic tape, or optical disk (the last three being located in disk and tape drives 158). Any suitable operating system and associated graphical user interface (e.g., Microsoft Windows, Linux) may direct CPU 151. In addition, computer 150 includes a control program 2517 that resides within computer memory storage 2516. Control program 2517 contains instructions that when executed on CPU 151 allow the computer 150 to carry out the operations described with respect to any of the methods of the present disclosure.

The computer 150 may be used in a computer based method for 3D digital endodontics, 3D imaging equipment being used to digitize an image of an infected tooth or teeth to thereby form image data. The 3D imaging equipment is for generation of volumetric data such as a CT scanner, an MRI scanner, or an ultrasound scanner. The user interface is preferably adapted to extract a 3D representation of a root canal system from the image data and visualized on a visual display unit. The user interface is preferably adapted to allow design of a surgical template to guide endodontic instruments to localize the root canal intra-operatively. The user interface is preferably adapted to provide a segmentation technique for obtaining a 3D description of the root canal system.

The user interface is preferably adapted to allow 3D and cross-sectional views of the root canal on coronal, sagittal and/or transverse plans.

For access via the user interface, digital templates of a plurality of root canal instruments are preferably provided, e.g. stored in memory or accessible via a telecommunications network such as a local area network or a wide area data network such as the Internet. The user interface co-operates with the digital templates of the plurality of root canal instruments to allow 2D superimposed views of instruments and canal shapes or canal cross-sections for treatment planning and instrument selection.

Software running on the computer system and is preferably provided for determining the location of root canal orifices by extracting the root canal system from the image data. This step of extracting can be carried out by indicating points along the axis of the root canal in one or multiple slices of the image. The points can be connected and make up a 3D line graph representative of the root canal system of the tooth.

The user interface and the software running on the computer system are preferably adapted to visualize the orifice of each root canal as a point with a distinct colour. Optionally the root canal orifice can e expressed as coordinates in a tooth specific coordinate system.

The user interface and the software running on the computer system are preferably adapted to visualize a surgical template and preferably the template is adapted to be supported on an occlusal surface of at least one of the infected teeth or adapted to be supported on an occlusal surface of one or more neighbouring teeth. Optionally the template is adapted to extend out onto gums and soft tissue.

The user interface and the software running on the computer system are preferably adapted to visualize in and to introduce into the template, one or more features to guide endodontic instruments used during a surgical intervention are introduced. For example, the one or more features can be guidance tubes that help direct the endodontic tools to the orifices of the root canals, thereby allowing for a less invasive intervention. Optionally, the one or more features may provide physical depth control to the endodontic instruments.

The user interface and the software running on the computer system can be adapted to provide the template with a flat or other surface on a non-supported side which acts as a physical stop for an endodontic instrument. The location of the physical stop can be determined by means of a calculation program running on the computer system which compares the length of the root canal as determined from the acquired 3D representation of the root canal and the length of the foreseen endodontic instrument, such that when inserted, the endodontic instrument penetrates the root canal at a desired depth. The endodontic instrument may be fitted with a flange to abut against said flat surface on the template. The flange can be hinged around a point on its longitudinal axis to assure a "face-to-face" connection at the interface with the template.

Optionally, the one or more of said features can be located at the level of the gums, oblique or perpendicular to the axis of the infected tooth.

The one or more features can be for guiding a dental bur or drill for making a lateral access hole through the bone surrounding the root of the infected tooth at the location of a lateral root canal that could not be cleaned via a typical occlusal access route through the pulp chamber.

The one or more features may allow for irrigation fluid to be injected into the root canal via an apical root end opening. The irrigation fluid may be designed to flow from the apical end of the root canal up to the pulp chamber and further out of the tooth via an occlusal access opening.

Those skilled in the art will appreciate that the hardware represented in Fig. 5 may vary for specific applications. For example, other peripheral devices such as optical disk media, audio adapters, or chip programming devices, such as PAL or EPROM programming devices well-known in the art of computer hardware, and the like may be utilized in addition to or in place of the hardware already described.

In the example depicted in figure 85, the computer program product for carrying out the method of the present disclosure can reside in any suitable memory. However, it is important that while the present disclosure has been, and will continue to be, that those skilled in the art will appreciate that the mechanisms of the present disclosure are capable of being distributed as a computer program product in a variety of forms, and that the present disclosure applies equally regardless of the particular type of signal bearing media used to actually carry out the distribution. Examples of computer readable signal bearing media include: recordable type media such as floppy disks and CD ROMs, solid state memories, tape storage devices, magnetic disks.

Accordingly, the present disclosure also includes a software product which when executed on a suitable computing device carries out any of the methods of the present disclosure.

Suitable software can be obtained by programming in a suitable high level language such as C and compiling on a suitable compiler for the target computer processor.

The present disclosure also provides a surgical template having at least one guide for an endodontic instrument for localizing a root canal intra-operatively. Having designed

the endodontic template, it can be manufactured by any suitable method such a numerically controlled milling, a rapid prototyping, or a layered manufacture system. In one preferred embodiment, Rapid Prototyping and Manufacturing (RP&M) techniques are used to manufacture the template. Rapid Prototyping and Manufacturing (RP&M) can be defined as a group of techniques used to quickly fabricate a scale model of an object typically using three-dimensional (3-D) computer aided design (CAD) data of the object. Currently, a multitude of Rapid Prototyping techniques is available, including stereo lithography (SLA), Selective Laser Sintering (SLS), Fused Deposition Modeling (FDM), foil-based techniques, etc.

A common feature of these techniques is that objects are typically built layer by layer. Stereo lithography, presently the most common RP&M technique, utilizes a vat of liquid photopolymer "resin" to build an object a layer at a time. On each layer, an electromagnetic ray, e.g. one or several laser beams which are computer-controlled, traces a specific pattern on the surface of the liquid resin that is defined by the two-dimensional cross-sections of the object to be formed. Exposure to the electromagnetic ray cures, or, solidifies the pattern traced on the resin and adheres it to the layer below. After a coat had been polymerized, the platform descends by a single layer thickness and a subsequent layer pattern is traced, adhering to the previous layer. A complete 3-D object is formed by this process.

Selective laser sintering (SLS) uses a high power laser or another focused heat source to sinter or weld small particles of plastic, metal, or ceramic powders into a mass representing the 3-dimensional object to be formed.

Fused deposition modelling (FDM) and related techniques make use of a temporary transition from a solid material to a liquid state, usually due to heating. The material is driven through an extrusion nozzle in a controlled way and deposited in the required place as described among others in U.S. Pat. No. 5.141.680.

Foil-based techniques fix coats to one another by means of gluing or photo polymerization or other techniques and cut the object from these coats or polymerize the object. Such a technique is described in U.S. Pat. No. 5.192.539.

Typically RP&M techniques start from a digital representation of the 3-D object to be formed, i.e. an endodontic template. Generally, the digital image is sliced into a series of cross-sectional layers which can be overlaid to form the object as a whole. The RP&M apparatus uses this data for building the object on a layer-by-layer basis. The cross-sectional data representing the layer data of the 3-D object may be generated using a computer system and computer aided design and manufacturing (CAD/CAM) software.

A selective laser sintering (SLS) apparatus is particularly preferred for the manufacture of the endodontic template from a computer model. It should be understood however, that various types of rapid manufacturing and tooling may be used for accurately fabricating these endodontic templates including, but not limited to, stereolithography (SLA), Fused Deposition Modeling (FDM) or milling.

The endodontic template may be manufactured in different materials. Preferably, only materials that are biocompatible with the human body are taken into account. In the case SLS is used as a RP&M technique, the endodontic template may be fabricated from a polyamide such as PA 2200 as supplied by EOS, Munich, Germany or Duraform PA from 3D Systems, South Caroline, USA, or any other material known by those skilled in the art may also be used.

It is also to be understood that the terminology used herein is for purposes of describing particular embodiments only, and is not intended to be limiting. It must be noted that the singular forms "a" "an" and "the" include singular and/or plural referents unless the context clearly dictates otherwise. It is also to be understood that plural forms include singular and/or plural referents unless the context clearly dictates otherwise. It is moreover to be understood that, in case parameter ranges are given which are delimited by numeric values, the ranges are deemed to include these limitation values.

The particular combinations of elements and features in the above detailed embodiments are exemplary only. As those skilled in the art will recognize, variations, modifications, and other implementations of what is described herein can occur to those of ordinary skill in the art.

Accordingly, the foregoing description is by way of example only and is not intended as limiting. The invention is defined in the following claims.

## Claims

1. A computer based method for 3D digital endodontics, 3D imaging equipment being used to digitize an image of an infected tooth or teeth to thereby form image data, the method comprising :
providing a user interface to extract a 3D representation of a root canal system from the image data and to visualize the root canal system on a display unit; **characterised by**
providing digital templates of a plurality of root canal instruments, allowing 2D superimposed views of instruments and canal shapes or canal cross-sections for treatment planning and instrument selection.

2. The method of claim 1 wherein a segmentation technique is provided for obtaining a 3D description of the root canal and wherein the user interface is adapted to allow 3D and cross-sectional views of the root canal on coronal, sagittal and/or transverse plans.

3. The method of any of the preceding claims wherein location of root canal orifices is determined by extracting the root canal system from the image data.

4. The method of claim 3, wherein the step of extracting is carried out by indicating points along the axis of the root canal in one or multiple slices of the image.

5. The method of claim 4, wherein the points are connected and make up a 3D line graph representative of the root canal system of the tooth.

6. The method of any of the claims 3 to 5, wherein the orifice of each root canal is visualized in 3D as a point with a distinct colour.

7. The method of any of the claims 3 to 6, wherein the root canal orifice is expressed as coordinates in a tooth specific coordinate system.

8. A computer based system for 3D digital endodontics, 3D imaging equipment being used to digitize an image of an infected tooth or teeth to thereby form image data, the system comprising :
a user interface to extract a 3D representation of a root canal system from the image data and to visualize the root canal system on a display unit and **characterised by** further comprising
digital templates of a plurality of root canal instruments stored in a memory, and
means for allowing 2D superimposed views of instruments and canal shapes or canal cross-sections for treatment planning and instrument selection

9. The system of claim 8, having
Means for gradient-based extraction of the root canal system out of the image data using the grey values and/or
Means to highlight the root canal in the image data, optionally including the side channels, following the use of a radiopaque dye which diffuses through the tissue in the root canal and the subsequent CBCT imaging, and/or
Means for automatic identification and tracing of the volume of the infected/inflamed tissue based on the grey values.

10. The system of any of the claims 8 and 9, further comprising means of segmentation for obtaining 3D description of the root canal system and wherein the user interface is adapted to allow 3D and cross- sectional views of the root canal on coronal, sagittal and/or transverse plans.

11. The system of any of the claims 8 to 10, further comprising means for location of root canal orifices by extracting the root canal system from the image data.

12. The system of claim 11, wherein the means for location is adapted to indicate points along the axis of the root canal in one or multiple slices of the image.

13. The system of claim 12, wherein the points are connected and make up a 3D line graph representative of the root canal system of the tooth.

14. The system of any of the claims 11 to 13, further comprising means for visualising the orifice of each root canal in 3D as a point with a distinct colour.

15. The system of any of the claims 11 to 14, further comprising means for expressing the root canal orifice as coordinates in a tooth specific coordinate system.

## Patentansprüche

1. Computerbasiertes Verfahren für digitale 3D-Endodontie, wobei eine 3D-Bildgebungsausrüstung zur Digitalisierung eines Bildes eines infizierten Zahns oder Zähne eingesetzt wird, um dadurch Bilddaten zu erzeugen, wobei das Verfahren Folgendes umfasst:
Bereitstellen einer Benutzerschnittstelle, um eine 3D-Darstellung eines Wurzelkanalsystems aus den Bilddaten zu extrahieren und das Wurzelkanalsystem auf einer Anzeigeeinheit sichtbar zu machen; **gekennzeichnet durch** Bereitstellen digitaler Schablonen einer Mehrzahl von Wurzel kanalinstrumenten,
welche überlagerte 2D-Ansichten von Instrumenten und
Kanalformen oder Kanalquerschnitten für die Behandlungsplanung und Instrumentenauswahl ermöglichen.

2. Verfahren nach Anspruch 1, wobei ein Segmentierungsverfahren zum Erhalten einer 3D-Darstellung des Wurzelkanals bereitgestellt wird und wobei die Benutzerschnittstelle dazu ausgelegt ist, 3D-und Querschnittsansichten des Wurzelkanals auf Koronal-, Sagittal- und/oder Transversal-Ebenen zu ermöglichen.

3. Verfahren nach einem beliebigen der vorangehenden Ansprüche, wobei die Position von Wurzelkanalöffnungen durch Extrahieren des Wurzelkanalsystems aus den Bilddaten ermittelt wird.

4. Verfahren nach Anspruch 3, wobei der Schritt des Extrahierens durch Anzeigen von Punkten entlang der Achse des Wurzelkanals in einer oder mehreren Schnitten des Bildes durchgeführt wird.

5. Verfahren nach Anspruch 4, wobei die Punkte verbunden werden und ein 3D-Liniendiagramm bilden, welches das Wurzelkanalsystem des Zahns darstellt.

6. Verfahren nach einem beliebigen der Ansprüche 3 bis 5, wobei die Öffnung jedes Wurzelkanals in 3D als ein Punkt mit einer ausgeprägten Farbe sichtbar gemacht wird.

7. Verfahren nach einem beliebigen der Ansprüche 3 bis 6, wobei die Wurzelkanalöffnung als Koordinaten in einem zahnspezifischen Koordinatensystem wiedergegeben wird.

8. Computerbasiertes System für digitale 3D-Endodontie, wobei eine 3D-Bildgebungsausrüstung zur Digitalisierung eines Bildes eines infizierten Zahns oder Zähne eingesetzt wird, um dadurch Bilddaten zu erzeugen, wobei das System Folgendes umfasst:
eine Benutzerschnittstelle, um eine 3D-Darstellung eines Wurzelkanalsystems aus den Bilddaten zu extrahieren und das Wurzelkanalsystem auf einer Anzeigeeinheit sichtbar zu machen, **dadurch gekennzeichnet, dass** es ferner Folgendes umfasst:
in einem Speicher gespeicherte digitale Schablonen einer Mehrzahl von Wurzelkanalinstrumenten, und
Mittel, um überlagerte 2D-Ansichten von Instrumenten und
Kanalformen oder Kanalquerschnitten für die Behandlungsplanung und Instrumentenauswahl zu ermöglichen.

9. System nach Anspruch 8, mit
Mitteln zur gradientenbasierten Extraktion des Wurzelkanalsystems aus den Bilddaten unter Verwendung der Grauwerte und/oder
Mitteln zum Hervorheben des Wurzelkanals in den Bilddaten, optional einschließlich der Seitenkanäle, nach Verwendung eines röntgendichten Farbstoffes, welcher durch das Gewebe im Wurzelkanal hindurch diffundiert, und anschließender CBCT-Bildgebung, und/oder
Mitteln zur automatischen Identifizierung und Ermittlung des Volumens des infizierten/entzündeten Gewebes auf der Grundlage der Grauwerte.

10. System nach einem beliebigen der Ansprüche 8 und 9, welches ferner Segmentierungsmittel zum Erhalt einer 3D-Darstellung des Wurzelkanalsystems umfasst und wobei die Benutzerschnittstelle dazu ausgelegt ist, 3D- und Querschnittsansichten des Wurzelkanals auf Koronal-, Sagittal- und/oder Transversal-Ebenen zu ermöglichen.

11. System nach einem beliebigen der Ansprüche 8 bis 10, welches ferner Mittel zur Ortsbestimmung von Wurzelkanalöffnungen durch Extrahieren des Wurzelkanalsystems aus den Bilddaten umfasst.

12. System nach Anspruch 11, wobei das Mittel zur Ortsbestimmung dazu ausgelegt ist, Punkte entlang der Achse des Wurzelkanals in einer oder mehreren Schnitten des Bildes anzuzeigen.

13. System nach Anspruch 12, wobei die Punkte verbunden werden und ein 3D-Liniendiagramm bilden, welches das Wurzelkanalsystem des Zahns darstellt.

14. System nach einem beliebigen der Ansprüche 11 bis 13, welches ferner Mittel zum Sichtbarmachen der Öffnung jedes Wurzelkanals in 3D als ein Punkt mit einer ausgeprägten Farbe umfasst.

15. System nach einem beliebigen der Ansprüche 11 bis 14, welches ferner Mittel zum Wiedergeben der Wurzelkanalöffnung als Koordinaten in einem zahnspezifischen Koordinatensystem umfasst.

## Revendications

1. Méthode assistée par ordinateur pour l'endodontie digitale en 3D, un équipement d'imagerie 3D étant utilisé pour digitaliser une image d'une ou plusieurs dent(s) infectée(s) pour ainsi former des données d'image, la méthode comprenant les étapes :
fournir une interface utilisateur pour extraire une représentation 3D d'un système de canaux radiculaires des données d'images et pour visualiser le système de canaux radiculaires sur une unité d'affichage ; **caractérisée par**
fournir des modèles digitaux d'une pluralité d'instruments pour canal radiculaire, permettre des vues superposées en 2D des instruments et des formes des canaux ou des sections transversales des canaux pour planifier un traitement ou la sélection des instruments.

2. Méthode selon la revendication 1, dans laquelle une technique de segmentation est fournie pour obtenir une description 3D des canaux radiculaires et dans laquelle l'interface utilisateur est adaptée pour permettre des vues en 3D et en coupe transversale des canaux radiculaires en plans coronal, sagittal et/ou transversal.

3. Méthode selon l'une des revendications précédentes, dans laquelle la position des orifices des canaux radiculaires est déterminée en extrayant le système de canal radiculaire des données d'image.

4. Méthode selon la revendication 3, dans laquelle l'étape d'extraction est mise en oeuvre en indiquant des points le long de l'axe des canaux radiculaires dans un ou plusieurs tranche(s) de l'image.

5. Méthode selon la revendication 4, dans laquelle les points sont connectés et forment un graphique linéaire en 3D représentatif du système de canaux radiculaires de la dent.

6. Méthode selon l'une des revendications 3 à 5, dans laquelle l'orifice de chaque canal radiculaire est visualisé en 3D comme un point avec une couleur distincte.

7. Méthode selon l'une des revendications 3 à 6, dans laquelle l'orifice de chaque canal radiculaire est exprimé comme coordonnées dans un système de coordonnées spécifique à la dent.

8. Système assisté par ordinateur pour l'endodontie digitale en 3D, un équipement d'imagerie 3D étant utilisé pour digitaliser une image d'une ou plusieurs dent(s) infectée(s) pour ainsi former des données d'image, le système comprenant :
une interface utilisateur pour extraire une représentation 3D d'un système de canaux radiculaires des données d'images et pour visualiser le système de canaux radiculaires sur une unité d'affichage, et **caractérisé par le fait qu'**il comprend encore :
des modèles digitaux d'une pluralité d'instruments pour canal radiculaire enregistrés dans une mémoire et,
des moyens pour permettre des vues superposées en 2D des instruments et des formes des canaux ou des sections transversales de canaux pour planifier un traitement ou la sélection des instruments.

9. Système selon la revendication 8, comprenant :
des moyens d'extraction sur la base du gradient du système de canaux radiculaires depuis les données d'image en utilisant des valeurs de gris et/ou
des moyens pour mettre en évidence les canaux radiculaires dans les données d'image, incluant de manière optionnelle les canaux latéraux, après l'utilisation d'une teinture radiopaque qui se répand à travers le tissu dans les canaux radiculaires et une imagerie CBCT ultérieure, et/ou
des moyens pour l'identification automatique et le tracé du volume des tissus enflammés/infectés basé sur les valeurs de gris.

10. Système selon l'une des revendications 8 et 9, comprenant encore des moyens de segmentation pour obtenir une description 3D du système de canaux radiculaires et dans lequel l'interface utilisateur est adaptée pour permettre des vues en 3D et en coupe transversale des canaux radiculaires en plans coronal, sagittal et/ou transversal.

11. Système selon l'une des revendications 8 à 10, comprenant encore des moyens de localisation des orifices des canaux radiculaires en extrayant le système de canaux radiculaires des données d'image.

12. Système selon la revendication 11, dans lequel les moyens de localisation sont adaptés pour indiquer des points le long de l'axe des canaux radiculaires dans une ou plusieurs tranche(s) de l'image.

13. Système selon la revendication 12, dans lequel les points sont connectés et forment un graphique linéaire en 3D représentatif du système de canaux radiculaires de la dent.

14. Système selon l'une des revendications 11 à 13, comprenant encore des moyens de visualisation de l'orifice de chaque canal radiculaire en 3D comme un point avec une couleur distincte.

15. Système selon l'une des revendications 11 à 14, comprenant encore des moyens pour exprimer l'orifice de chaque canal radiculaire comme coordonnées dans un système de coordonnées spécifique à la dent.
